# EUROPÄISCHE PATENTANMELDUNG

(11) **EP 4 491 162 A1**
(43) Veröffentlichungstag der Anmeldung: **15.01.2025**
(21) Anmeldenummer: 23184565.2
(22) Anmeldetag: 10.07.2023
(51) Int. Cl.: A61F 7/00

(54) **EINE TRAGBARE KALT- UND WÄRMEVORRICHTUNG**

(71) Anmelder: Xeno Patch UG, 82234 Wessling (DE)
(72) Erfinder: Thouet, Paul, 80807 München (DE); Schmid, Elias, 80686 München (DE)

(57) **Zusammenfassung**

Die Erfindung betrifft eine tragbare Kalt- und Wärmevorrichtung (10) zum Temperieren einer Körperstelle, aufweisend einen Gurt (12) bestehend aus mindestens zwei im Wesentlichen zumindest teilweise übereinanderliegenden Lagen (12a, 12b) und mindestens ein thermoelektrisches Modul (14a-n) aufweisend mindestens ein thermoelektrisches Element (141), vorzugsweise ein Peltierelement. Des Weiteren weist die Vorrichtung (10) mindestens einen Lüfter (16a-n), vorzugsweise einen Radiallüfter, auf. Der Gurt (12) weist eine erste Lage (12a), die der Körperstelle zugewandt ist, und eine zweite Lage (12b), die der Körperstelle abgewandt ist, auf. Das mindestens eine thermoelektrische Modul (14a-n) und/ oder der Lüfter (16a-n) sind zumindest an die erste Lage (12a) und/ oder zweite Lage (12b) befestigt, wobei das thermoelektrische Modul (14a-n) und der Lüfter (16a-n) voneinander getrennt an dem Gurt (12) angeordnet sind.

## Beschreibung

### Technisches Gebiet

Die vorliegende Erfindung betrifft eine tragbare Kalt- und Wärmevorrichtung zum Temperieren einer Körperstelle.

### Stand der Technik

Die Anwendung von Kalt- und Wärmetherapien zur Behandlung verschiedener Erkrankungen und Verletzungen ist ein bekanntes Verfahren in der medizinischen Praxis. Bei solchen Therapien ist es von entscheidender Bedeutung, dass die Kälte- oder Wärmequelle eng an die zu behandelnde Körperstelle angelegt wird, um eine optimale Wirksamkeit zu gewährleisten. Eine vielversprechende Technologie, die für solche Therapien eingesetzt wird, sind thermoelektrische Module mit Peltierelementen.

Peltierelemente sind elektronische Bauelemente, die in der Lage sind, Wärme von einer Seite auf die andere zu übertragen, je nach der Richtung des elektrischen Stroms, der durch sie hindurchfließt. Sie können sowohl als Kältequelle als auch als Wärmequelle fungieren und somit für Kalt- und Wärmetherapien genutzt werden. Obwohl die Verwendung von Peltierelementen für therapeutische Zwecke bekannt ist, stellt die effektive Anwendung eine Herausforderung dar. Um eine maximale therapeutische Wirkung zu erzielen, müssen die Peltierelemente eng an die zu behandelnde Körperstelle angelegt werden. Dies stellt sicher, dass die Kälte oder Wärme effizient auf das Gewebe übertragen wird und die gewünschten therapeutischen Effekte erzielt werden.

In Anbetracht dieser Herausforderung gibt es einen Bedarf an innovativen Lösungen, die es ermöglichen, Peltierelemente so zu positionieren, dass sie eng an die zu behandelnde Körperstelle anliegen. Eine effektive und komfortable Anwendung von Kalt- und Wärmetherapien kann somit gewährleistet werden, was zu einer verbesserten Behandlung und schnelleren Genesung der Patienten führt.

Die Patentschrift WO2022133209A1 beschreibt eine temperatursteuerbare Wickelanordnung. Ein erstes Temperatursteuermodul umfasst ein Gehäuse, ein steuerbares Temperaturelement, ein Spreizelement und mindestens einen ersten Fingerspreizer, der schwenkbar am Spreizelement befestigt ist. Dabei ist eine untere Oberfläche des Spreizelements so positioniert, dass diese das Körperteil des Benutzers berührt.

Es besteht jedoch stets Bedarf, Therapiegurte flexibler und komfortabler zu gestalten, wobei gleichzeitig der direkte Kontakt zwischen der zu behandelnden Körperstelle und dem thermoelektrischen Modul aufrechterhalten werden soll. Herkömmliche Therapiegurte sind oft sperrig und unflexibel, was zu einer eingeschränkten Bewegungsfreiheit und Unannehmlichkeiten für den Benutzer führen kann.

Die Herausforderung besteht darin, eine Vorrichtung zu entwickeln, die es ermöglicht, die Vorteile der Kalt- und Wärmetherapie in einer komfortablen und flexiblen Weise zu vereinen. Dies erfordert, dass die Wärmeenergie direkt an die Körperstelle übertragen wird, ohne den Kontakt durch weitere Elemente wie Spreizfinger zu stören.

Durch die Schaffung eines flexibleren und komfortableren Therapiegurtes, der eine direkte Übertragung der Wärmeenergie auf die zu behandelnde Körperstelle ermöglicht, wird eine effektive und angenehme Behandlung gewährleistet. Dies kann dazu beitragen, Schmerzen zu lindern, Entzündungen zu reduzieren und die Genesung zu fördern, während der Benutzer gleichzeitig einen verbesserten Tragekomfort und eine größere Bewegungsfreiheit genießt.

### Darstellung der Erfindung

Ausgehend von dem bekannten Stand der Technik ist es eine Aufgabe der vorliegenden Erfindung, eine verbesserte Lösung für eine tragbare Kalt- und Wärmevorrichtung anzugeben, welche die Flexibilität der Vorrichtung für die Anpassung an eine zu behandelnde Körperstelle erhöht und zugleich kompakt und flach ausgeführt werden kann.

Diese Aufgabe wird durch ein Kalt- und Wärmevorrichtung mit den Merkmalen des Anspruchs 1 gelöst. Vorteilhafte Weiterbildungen ergeben sich aus den Unteransprüchen, der vorliegenden Beschreibung sowie den Figuren.

Entsprechend wird eine tragbare Kalt- und Wärmevorrichtung zum Temperieren einer Körperstelle vorgeschlagen, welche einen Gurt bestehend aus mindestens zwei im Wesentlichen zumindest teilweise übereinanderliegenden Lagen, mindestens ein thermoelektrisches Modul aufweisend mindestens ein thermoelektrisches Element, vorzugsweise ein Peltierelement, und mindestens einen Lüfter, vorzugsweise einen Radiallüfter aufweist. Der Gurt weist eine erste Lage, die der Körperstelle zugewandt ist, und eine zweite Lage, die der Körperstelle abgewandt ist, auf. Das mindestens eine thermoelektrische Modul und/ oder der Lüfter sind zumindest an die erste Lage und/ oder zweite Lage befestigt. Dabei sind das thermoelektrische Modul und der Lüfter voneinander getrennt an dem Gurt angeordnet.

Es wurde erkannt, dass die Flexibilität des Gurtes erhöht werden kann, indem das thermoelektrische Modul und der Lüfter im Vergleich zu bekannten Ausführungsformen im Stand der Technik voneinander getrennt auf dem Gurt angeordnet werden kann. Dies hat den Vorteil, dass der Lüfter die durch Kühlung des thermoelektrischen Elements aufgewärmte Luft nicht wieder einsaugt. In anderen Worten ist durch die räumlich getrennte Anordnung des Lüfters und der Auslassöffnung sichergestellt, dass die Einlassöffnung des Lüfters und die Auslassöffnung ausreichenden Abstand voneinander haben. Dadurch wird gewährleistet, dass der aus den Auslassöffnungen austretendende aufgewärmte Luftstrom nicht in fluider Verbindung mit der Einlassöffnung des Lüfters steht. Dies führt zu einer verbesserten die Kühlung des thermoelektrischen Elements, vorzugsweise Peltierelements, und somit kann die Effizienz der Kalt- und Wärmevorrichtung weiter verbessert werden.

Des Weiteren kann die Vorrichtung flacher ausgestaltet werden, da der Lüfter nicht auf dem thermoelektrischen Modul angeordnet ist. Dadurch kann der Tragekomfort für den Benutzer signifikant erhöht werden. Es ermöglicht beispielsweise ein Tragen des Gurtes unter der Kleidung im Alltag.

Durch die Gestaltung des Therapiegurtes in einer Weise, die eine direkte Übertragung der Wärmeenergie auf die Körperstelle ermöglicht, kann eine effiziente und gezielte Therapie gewährleistet werden. Dies hat den zusätzlichen Vorteil, dass die Vorrichtung flacher ausgestaltet werden kann, was den Tragekomfort weiter verbessert.

Ein Gurt kann auch als Wickelanordnung oder Band bezeichnet werden und ist zu verstehen als eine flexible und längliche Vorrichtung, die verwendet wird, um die Vorrichtung fest um eine Körperstelle zu wickeln, zu fixieren oder zu umschließen.

In der hier beschriebenen Anwendung kann der Gurt auch als ein Therapiegurt bezeichnet werden. Dementsprechend werden Kalt- und Wärmetherapiegeräte wie z.B. Peltierelemente an den Therapiegurt befestigt, um die Geräte in Kontakt mit einer zu behandelnden Körperstelle zu bringen.

Vorzugsweise ist das thermoelektrische Modul und der Lüfter derart zueinander ausgerichtet, dass diese entlang einer Gerade quer zur Längsrichtung oder Umfangsrichtung des Gurtes angeordnet sind. Dabei ist der Lüfter nicht wie im Stand der Technik auf dem thermoelektrischen Modul angeordnet, sondern an dem Gurt im Wesentlichen auf der gleichen Ebene wie das thermoelektrische Modul angeordnet.

Die Längsrichtung oder Umfangsrichtung bezeichnet dabei die Richtung, die durch die Wicklung des Gurtes um eine Körperstelle definiert wird.

In einem Beispiel sind das mindestens eine thermoelektrische Modul und der mindestens eine Lüfter über die Vorderseite, welche der Körperstelle zugewandt ist, an die erste Lage und/ oder über die Rückseite, welche der Körperstelle abgewandt ist, an die zweite Lage befestigt.

Das thermoelektrische Modul weist im vorderen Bereich das thermoelektrische Element, vorzugsweise Peltierelement, auf. Der vordere Bereich ist als der Bereich zu verstehen, der einer Körperstelle im angebrachten Zustand zugewandt ist. In einem Beispiel ist die Oberseite des thermoelektrischen Elements, vorzugsweise Peltierelement, direkt mit der Körpestelle in Kontakt. Die Oberseite ist als die Seite zu verstehen, die mit der Körperstelle in Kontakt steht und die Wärmeenergie an eine zu behandelnde Körperstelle überträgt. Beispielsweise besteht die Oberseite der Peltierlements aus Aluminiumoxid Keramik.

In einem weiteren Beispiel ist an der Oberseite des thermoelektrischen Elements eine Materialschicht auf dem thermoelektrischen Element aufgebracht. Die Materialschicht kann beispielsweise aus einer Aluminiumplatte bestehen.

Ein Peltierelement, auch bekannt als Peltier-Kühler oder Peltier-Modul, ist ein thermoelektrisches Gerät, das nach dem Peltier-Effekt arbeitet. Es besteht aus Halbleitermaterialien, normalerweise Bismuttellurid, die zu einer Sandwichstruktur zusammengefügt sind. Die Halbleiterschichten sind mit elektrischen Kontakten verbunden.

Der Peltier-Effekt tritt auf, wenn ein Strom durch das Peltierelement fließt. Dadurch entsteht ein Temperaturunterschied an den beiden Seiten des Elements. An einer Seite wird Wärme absorbiert, während an der anderen Seite Wärme abgegeben wird. Dies ermöglicht die Erzeugung eines Temperaturdifferentials.

Die Kühlung von thermoelektrischen Elementen kann durch Kühlkörper, vorzugsweise Wärmesenken, gewährleistet werden

Der Lüfter wird eingesetzt, um einen Luftstrom über einen Kühlkörper zu erhöhen und die Kühlleistung zu verbessern. Der Lüfter führt die Wärme ab, indem er die umgebende Luft über den Kühlkörper bläst und so die Kühlung des thermoelektrischen Elements unterstützt.

Um die Wärmeübertragung zwischen dem Peltierelement und dem Kühlkörper zu verbessern, kann eine Wärmeleitpaste verwendet werden. Die Wärmeleitpaste füllt etwaige Lücken und Luftblasen zwischen dem thermoelektrischen und dem Kühlkörper aus und erhöht so die Kontaktfläche und die Effizienz der Wärmeübertragung.

Neben Peltierelementen können auch alternativ kalorische Materialien wie magnetokalorische und elektrokalorische Materialien zum Einsatz kommen. Diese Materialien nutzen die kalorischen Effekte, um Wärmeenergie zu erzeugen oder abzuführen.

Das magnetokalorische Prinzip beruht auf der Änderung der magnetischen Eigenschaften eines Materials unter Einfluss eines externen Magnetfeldes. Durch das Anlegen oder Entfernen eines Magnetfeldes kann das Material Wärme aufnehmen oder abgeben. Dieser Effekt ermöglicht die Kontrolle der Temperatur in der Nähe der zu behandelnden Körperstelle, ohne den direkten Kontakt zu beeinträchtigen.

Das elektrokalorische Prinzip hingegen nutzt die Änderung der elektrischen Feldstärke, um Temperaturänderungen in einem Material zu erzeugen. Durch Anlegen oder Entfernen einer elektrischen Spannung wird Wärmeenergie erzeugt oder abgeführt.

Der Einsatz kalorischer Materialien als Alternative zu Peltierelementen ermöglicht eine vielseitige Anpassung und Gestaltung von Therapievorrichtungen. Sie bieten die Möglichkeit, kompaktere und effizientere Vorrichtungen zu entwickeln, bei denen die Wärmeenergie direkt und gezielt an die zu behandelnde Körperstelle übertragen werden kann. Dies kann den Tragekomfort verbessern und die therapeutischen Effekte optimieren. Vorzugsweise weist die Kalt- und Wärmevorrichtung eine Kontrolleinrichtung und eine Batterieeinrichtung auf, um das thermoelektrische Modul und den Lüfter zu betreiben. Die Kontrolleinrichtung kann beispielsweise derart konfiguriert sein, um die Behandlung in Bezug auf Dauer, Temperaturbereiche und Zyklen zu steuern.

In einem ersten Beispiel können das thermoelektrische Modul und/ oder der Lüfter lediglich an die erste Lage befestigt sein. Dieser Aufbau gewährleistet maximale Flexibilität.

In einem weiteren Beispiel können das thermoelektrische Modul und/ oder der Lüfter an die erste Lage und an die zweite Lage befestigt sein. Dies gewährleistet eine gute Ausrichtung des thermoelektrischen Moduls und des Lüfters zueinander. Dadurch kann die Kühlung des thermoelektrischen Moduls optimiert werden.

In einer alternativem Ausführungsform kann das thermoelektrische Modul und/ oder der Lüfter lediglich an die zweite Lage befestigt sein. Dies gewährleistet ebenfalls eine hohe Flexibilität. Des Weiteren wird das thermoelektrische Modul durch die zweite Lage an die Körperstelle angepresst, da die zweite Lage außen liegt und das thermoelektrische Modul an die Körperstelle über eine Rückseite des thermoelektrischen Moduls auf die Körperstelle spannt. Die Rückseite ist als die Seite der Vorrichtung zu verstehen, die einer Körperstelle abgewandt ist, wenn die Vorrichtung an einer Körperstelle angebracht oder umgewickelt ist.

Ein Radiallüfter ist eine Art von Ventilator, der Luft oder Gas radial von der Mitte des Lüfters nach außen befördert. Im Gegensatz zu Axiallüftern, bei denen die Luft parallel zur Drehachse des Lüfters strömt, wird bei Radiallüftern die Luft senkrecht zur Drehachse bewegt. Radiallüfter sind auch unter dem Begriff "Radialventilator" oder "Zentrifugallüfter" bekannt.

Ein Radiallüfter besteht typischerweise aus einem Gehäuse, in dem sich ein Laufrad oder Impeller befindet. Das Laufrad besteht in einem Beispiel aus gekrümmten Schaufeln, die radial angeordnet sind. Wenn das Laufrad rotiert, erzeugt es eine Zentrifugalkraft, die die Luft von der Mitte des Lüfters nach außen drückt. Die Luft strömt dann durch das Gehäuse und wird seitlich oder nach vorne ausgestoßen. Die Nutzung von Radiallüftern ermöglicht eine besonders flache Ausführung der Vorrichtung im Gegensatz zu Axiallüftern.

In einem Beispiel weist das thermoelektrische Modul ein thermoelektrisches Element, vorzugsweise ein Peltierelement, einen Kühlkörper, vorzugsweise eine Wärmesenke und einen Rahmen auf.

Gemäß einer Ausführungsform ist der Lüfter derart ausgerichtet ist, um einen Luftstrom zwischen der ersten und zweiten Lage zu erzeugen. In einem Beispiel kann der Lüfter als Radiallüfter ausgestaltet sein und die Einlassöffnung des Radiallüfter an der ersten und/ oder zweiten Lage angeordnet sein. In einem weiteren Beispiel kann der Lüfter als Axiallüfter ausgestaltet sein und die Einlassöffnung des Axiallüfters zwischen der ersten und zweiten Lange angeordnet sein. Dadurch kann eine direkte Kühlluftströmung an das thermoelektrische Modul, insbesondere an die wärmeableitenden Elemente wie Wärmesenken, realisiert werden.

Der Raum zwischen der ersten und zweiten Lage ist im Wesentlichen durch die Höhe des thermoelektrischen Moduls und den Lüfter definiert.

Zudem hat eine Anordnung der Einlassöffnung des Lüfters zwischen der ersten und zweiten Lage bzw. auf den Stirnseiten der Vorrichtung den Vorteil, dass die Luft besser eingesaugt werden kann, wenn ein Benutzer die Vorrichtung unter der Kleidung trägt.

Gemäß einer Ausführungsform ist eine Auslassöffnung luftstromabwärts angeordnet, wobei die Auslassöffnung in der zweiten Lage oder zwischen der ersten und zweiten Lage angeordnet ist.

Dabei ist die Auslassöffnung luftstromabwärts nach dem thermoelektrischen Modul angeordnet.

Dabei bezeichnet luftstromabwärts die Luftstromrichtung von Lüfter zum Auslass, wobei das thermoelektrische Modul im Wesentlichen zwischen Lüfter und Auslassöffnung angeordnet ist.

Eine Anordnung der Auslassöffnung zwischen der ersten und zweiten Lage hat den Vorteil, dass die Wärme des thermoelektrischen Moduls besser abgeleitet werden kann, wenn der Benutzer die Vorrichtung unter der Kleidung trägt.

In einer weiteren beispielhaften Ausführungsform ist der mindestens eine Lüfter, das mindestens eine thermoelektrische Modul und die mindestens eine Auslassöffnung entlang einer Gerade angeordnet. Dadurch kann die Flexibilität des Gurtes weiter verbessert werden.

Vorzugsweise weist die erste Lage einen Temperaturübertragungsbereich auf, in welchem die Temperaturenergie des mindestens einen thermoelektrischen Moduls auf eine zu behandelnde Körperstelle übertragbar ist und einen Befestigungsbereich auf, wobei der Befestigungsbereich derart konfiguriert ist, um den Gurt an eine Körperstelle zu befestigen. Der Gurt wird, nachdem dieser um die Körperstelle gewickelt wurde, befestigt, um einen sicheren Halt zu gewährleisten. Er kann eine variable Länge haben und kann eine einstellbare Verschlusstechnik wie Klettverschluss, Schnallen oder Haken- und/ oder Ösenverschlüsse aufweisen, um die Festigkeit und den Sitz des Gurts anzupassen.

Gemäß einer Ausführungsform ist eine Breite des Lüfters in Bezug auf die Umfangsrichtung des Gurtes um -5% bis +50%, vorzugsweise -5% bis +25%, besonders bevorzugt -5% bis +5% im Vergleich zu der Breite des thermoelektrischen Moduls dimensioniert.

Dadurch wird sichergestellt, dass die Flexibilität des Gurtes nicht durch den Lüfter beschränkt wird. Vorzugsweise ist die Breite des Lüfters in etwa gleich groß dimensioniert, sodass die Flexibilität des Gurtes beim Anbringen bzw. Umwickeln an eine Körperstelle durch die Breite des thermoelektrischen Moduls und die Flexibilität bestimmt wird.

Die Breite des Lüfters und des thermoelektrischen Moduls ist die Länge, welche in Längsrichtung oder Umlaufrichtung des Gurtes entlang verläuft.

Gemäß einer besonders bevorzugten Ausführungsform sind die Mittelpunkte des thermoelektrischen Moduls und des Lüfters im Wesentlichen auf einer Gerade, welche vorzugsweise quer zur Umfangsrichtung angeordnet ist, zueinander ausgerichtet. Dadurch wird die Flexibilität des Gurtes weiter verbessert, da der Lüfter und das thermoelektrische Modul optimal aufeinander ausgerichtet sind. Dies hat weiterhin den Vorteil, dass das thermoelektrische Modul optimal zu dem Lüfter ausgerichtet ist und somit die Kühlung maximal auf das thermoelektrische Element gerichtet ist.

Gemäß einer Ausführungsform weisen die erste Lage und/ oder die zweite Lage des Gurtes Ausnehmungen auf, um das thermoelektrische Modul und/ oder den Lüfter an den Gurt zu anzuordnen.

Dadurch kann die Oberseite des thermoelektrischen Moduls direkt an die Körperstelle angebracht werden. Weiterhin kann dadurch die Flexibilität des Gurtes angepasst werden.

In einem Beispiel sind das thermoelektrische Modul an die erste Lage und/oder zweite Lage des Gurtes stoffschlüssig, vorzugsweise geklebt, und/ oder formschlüssig, vorzugsweise durch einen Druckknopfverschluss befestigt, und/ oder der Lüfter an die erste Lage und/oder zweite Lage des Gurtes geklebt und/ oder über einen Druckknopfverschluss befestigt.

Vorzugsweise weist die erste Lage eine geringere Knicksteifigkeit als die zweite Lage auf. Dies hat den Vorteil, dass sich die thermoelektrischen Elemente, vorzugsweise Peltierelemente, besser an die zu behandelnde Körperstelle anschmiegen können.

Als Flexibilität kann auch geringe Knicksteifigkeit verstanden werden.

In einem Beispiel besteht die erste Lage aus einem wasserundurchlässigen beschichteten Material, vorzugsweise Kunstleder oder einem Stoffgewebe mit einer Polyurethan (PU)-Schaum-Beschichtung. Dies hat den Vorteil, dass der Tragekomfort verbessert wird, da Flüssigkeit, wie Körperflüssigkeit, z.B. Schweiß des Benutzers, nicht durch den Gurt aufgenommen wird.

Gemäß einer Ausführungsform sind eine Reihe von thermoelektrischen Modulen und Lüfter mit jeweiligen Auslassöffnungen entlang der Umfangsrichtung des Gurtes in einem Temperaturübertragungsbereich in einem Abstand von mindestens 0,1cm bis höchstens 10cm, vorzugsweise 1cm - 3cm angeordnet.

Es wurde festgestellt, dass die Anordnung mehrerer kleiner thermoelektrischer Module entlang des Umfangs des Gurtes den Flexibilitätsgrad des Gurtes erhöht, insbesondere im Temperaturübertragungsbereich. Eine Breite von 20-50mm, vorzugsweise 30-40mmmm für die Oberseite des thermoelektrischen Elements, insbesondere eines Peltierelements, wird vorgeschlagen. Es wurde gezeigt, dass dies einen optimalen Kompromiss ermöglicht, indem einerseits die Wärmeenergie effektiv an die Körperstelle übertragen wird und gleichzeitig die Flexibilität des Gurtes gewährleistet wird.

Gemäß einer weiteren Ausführungsform sind mindestens ein weiterer Lüfter an jeweils einem Ende des Temperaturübertragungsbereich entlang der Umfangsrichtung angeordnet. Dadurch kann die Kühlung der thermoelektrischen Module erhöht werden, wobei gleichzeitig die Vorrichtung flacher ausgestaltet werden kann als bei einer Anordnung eines Lüfters, das auf dem thermoelektrischen Modul in Höhenrichtung angeordnet ist.

Gemäß einer bevorzugen Ausführungsform weist die Vorrichtung eine Spannvorrichtung auf, sodass der Gurt im Temperaturübertragungsbereich an die Körperstelle anpressbar ist.

Dadurch kann der Kontakt des thermoelektrischen Elements, vorzugsweise Peltierelements an die zu behandelnde Körperstelle verbessert werden und somit die Wärmeenergie des thermoelektrischen Elements in effizienterer Weise an die zu behandelnde Körperstelle übertragen werden.

Die Spannvorrichtung ist in einem Beispiel an der Außenseite angebracht. Die Außenseite ist eine Seite des Gurtes, die von der zu behandelnden Körperstelle abgewandt ist bzw. weg weist.

Gemäß einer anderen Ausführungsform weist die Spannvorrichtung Distanzelemente auf, um den Anpressdruck entlang des Gurtes anzupassen.

In einem Beispiel können die Distanzelemente zwischen Spanngurt und der Rückseite des thermoelektrischen Moduls angeordnet sein. In einem weiteren Beispiel können die Distanzelemente entlang der Spannvorrichtung verschiebbar angeordnet sein, um den Anpressdruck der thermoelektrischen Elemente auf die Körperstelle anzupassen. Insbesondere können Distanzelemente derart zwischen der Rückseite des thermoelektrischen Elements und der Spannvorrichtung positioniert werden, sodass die in der Mitte positionierten thermoelektrischen Elemente mit dem gleichen Anpressdruck an die Körperstelle gedrückt werden wie die außenliegenden thermoelektrische Elemente. Diese ist beispielsweise der Fall, wenn Gurt bzw. der Temperaturübertragungsbereich an eine gewölbte Körperstelle angebracht ist.

Die Distanzelemente können beispielswiese als Luftkissen, Schaumstoff oder Gummipad ausgestaltet sein.

In einem weiteren Beispiel können die Distanzelemente auch durch unterschiedliche Höhen der Kühlkörper, vorzugsweise Wärmesenken der thermoelektrischen Module entlang der Umlaufrichtung des Gurtes ausgestaltet sein. Die Höhe der Wärmesenken können beispielsweise derart ausgestaltet sein, dass die Höhe der Wärmesenken der thermoelektrischen Module in der Mitte des Temperaturübertragungsbereichs länger ausgestaltet sind als die Wärmesenken an den Rändern des Temperaturübertragungsbereichs. Dadurch kann sichergestellt werden, dass die thermoelektrischen Elemente gleichmäßig an einer zu behandelnden Körperstelle anliegen.

Gemäß einer weiteren Ausführungsform weist die Kälte und Wärmevorrichtung eine Temperaturverteilerlage auf, welche an einer der Körperstelle zugewandten Seite der ersten Lage des Gurtes befestigbar ist. Dies ermöglicht eine breite und gleichmäßige Verteilung der Wärmeenergie des thermoelektrischen Moduls auf die Temperaturverteilerlage und somit die zu behandelnde Körperstelle. Die Temperaturverteilerlage kann auch als flexible Wärmeübertragungsschicht bezeichnet werden und kann aus einem wärmeleitenden Polymer, z.B. Wärmeleitsilikon, oder wärmeleitenden TPU bestehen.

Gemäß einer weiteren Ausführungsform weist das thermoelektrische Element mindestens zwei Peltierelemente auf. Dadurch kann die erzeugbare Wärmeenergie des thermoelektrischen Moduls erhöht werden und höhere Temperaturunterschiede der Kalt- und Wärmezyklen und somit eine höhere Effizienz erzielt werden.

Gemäß einer weiteren Ausführungsform weist das thermoelektrische Modul ferner eine Wärmesenke auf, welche durch einen Rahmen an das thermoelektrische Element fixiert wird.

Dadurch wird das thermoelektrische Modul von Stößen geschützt und mechanische Spannungen zwischen den Bauteilen des thermoelektrischen Moduls kompensiert. Dadurch kann die Haltbarkeit der Vorrichtung erhöht werden.

Gemäß einer weiteren Ausführungsform weist die Vorrichtung ferner eine Platte auf, welche auf die zweite Lage von außen aufgebracht ist und über eine Schraubverbindung mit der Wärmesenke des thermoelektrischen Moduls verbunden ist. Dadurch kann der Halt der thermoelektrischen Module an den Gurt erhöht werden.

Gemäß einer weiteren Ausführungsform weist die Vorrichtung flexible Luftstromleitplanken auf, welche einen Luftstromkanal vom Lüfter zum thermoelektrischen Modul bilden. Dadurch kann die Kühlung des thermoelektrischen Moduls verbessert werden. Die Luftstromleitplanken können aus einem luftundurchlässigen flexiblen Material, beispielsweise Gummi, bestehen.

### Kurze Beschreibung der Figuren

Bevorzugte weitere Ausführungsformen der Erfindung werden durch die nachfolgende Beschreibung der Figuren näher erläutert. Dabei zeigen:
- Figur 1A-C: eine schematische Ansicht der Vorder- und Rückseite sowie eine Seitenansicht der tragbaren Kalt- und Wärmevorrichtung gemäß einer Ausführungsform;
- Figur 2A-B: schematische Schnittansichten der tragbaren Kalt- und Wärmevorrichtung gemäß einer Ausführungsform;
- Figur 3: eine schematische Rückseitenansicht der tragbaren Kalt- und Wärmevorrichtung mit seitlich angeordneten Lüftern gemäß einer weiteren Ausführungsform;
- Figur 4: eine schematische Schnittansicht der tragbaren Kalt- und Wärmevorrichtung mit einer Spannvorrichtung gemäß einer Ausführungsform;
- Figur 5: eine schematische Schnittansicht der tragbaren Kalt- und Wärmevorrichtung mit einer Temperaturverteilerlage gemäß einer Ausführungsform;
- Figur 6: eine schematische perspektivische Ansicht des thermoelektrischen Moduls gemäß einer Ausführungsform;
- Figur 7: eine schematische Schnittansicht des thermoelektrischen Moduls mit einer Platte zur Befestigung des thermoelektrischen Moduls an die zweite Lage gemäß einer Ausführungsform; und
- Figur 8A-C: eine schematische perspektivische Vorder- und Rückseitenansicht und eine schematische Schnittansicht der tragbaren Kalt- und Wärmevorrichtung gemäß einer weiteren Ausführungsform mit mindestens einem Axiallüfter.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

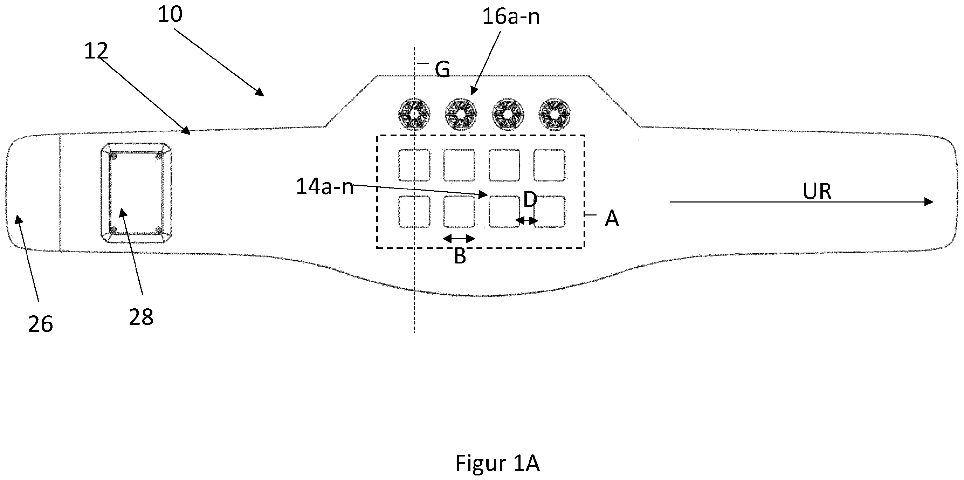

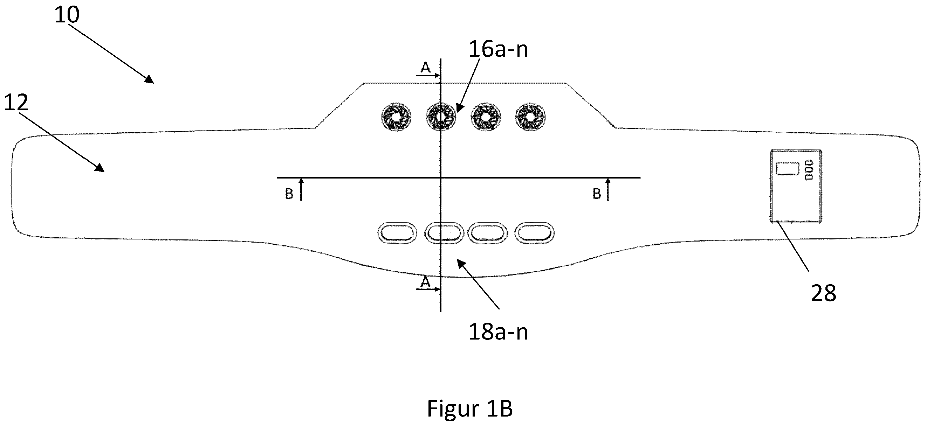

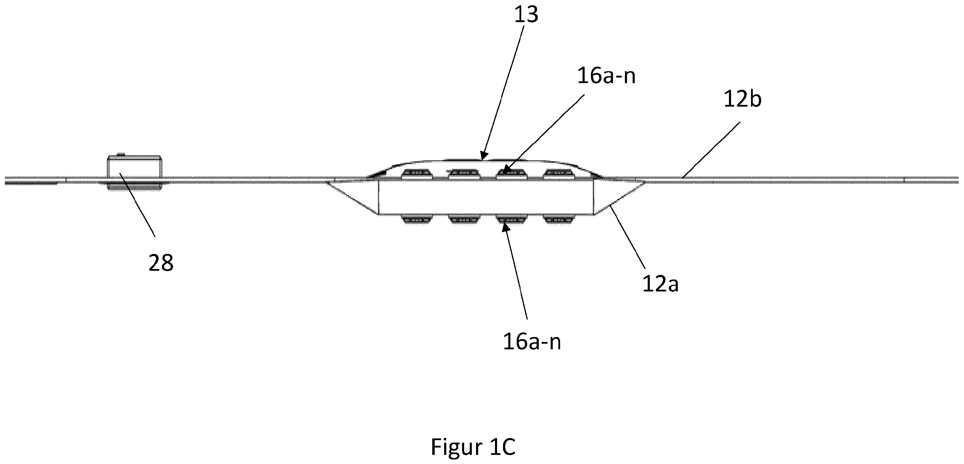

Figur 1A zeigt eine Vorderseite und Figur 1B eine Rückseite einer tragbaren Kalt- und Wärmevorrichtung 10 zum Temperieren einer Körperstelle, aufweisend einen Gurt 12 bestehend aus mindestens zwei im Wesentlichen zumindest teilweise übereinanderliegenden Lagen 12a, 12b (siehe Figur 1C) und mindestens ein thermoelektrisches Modul 14a-n aufweisend mindestens ein thermoelektrisches Element 141, vorzugsweise ein Peltierelement. Weiterhin weist die Vorrichtung mindestens einen Lüfter 16a-n, vorzugsweise einen Radiallüfter, auf. Der Gurt 12 weist eine erste Lage 12a, die der Körperstelle zugewandt ist, und eine zweite Lage 12b, die der Körperstelle abgewandt ist, auf. Das mindestens eine thermoelektrische Modul 14a-n und/ oder der Lüfter 16a-n sind zumindest an die erste Lage 12a und/ oder zweite Lage 12b befestigt, wobei das thermoelektrische Modul 14a-n und der Lüfter 16a-n voneinander getrennt an dem Gurt 12 angeordnet sind.

Weiterhin weist die Vorrichtung eine Kontrolleinrichtung 28 auf, um die Vorrichtung zu steuern beziehungsweise die Vorrichtung 10 in Bezug auf verschiedene Parameter wie Temperaturbereiche, Anzahl und Dauer der Kalt und Wärmezyklen und Dauer der Therapie zu steuern.

Wie in Figur 1A gezeigt, weist der Gurt 12 einen Befestigungsbereich 26 (z.B. Klettverschluss) auf, um diesen, nachdem es um eine Körperstelle (nicht gezeigt) gebunden oder gewickelt wurde, zu fixieren.

In dem hier gezeigten Beispiel sind acht thermoelektrische Module 14a-n in jeweils zwei in Umfangsrichtung UR parallel verlaufende Reihen mit jeweils vier thermoelektrischen Modulen 14a-n angeordnet. Der Temperaturübertragungsbereichs A ist durch die Anordnung der thermoelektrischen Module 14a-n definiert.

Des Weiteren ist hier beispielhaft gezeigt, dass das mindestens eine thermoelektrische Modul 14a-n und der mindestens eine Lüfter 16a-n auf einer Gerade G quer zur Umfangsrichtung UR gelegen sind.

Durch diese Anordnung kann eine maximale Flexibilität des Gurtes 12 erreicht werden, da diese durch die Breite B des thermoelektrischen Moduls 14a-n bestimmt wird. In anderen Worten ist die Breite B des hier beispielhaften gezeigten Radiallüfters kleiner oder gleich groß wie die Breite des thermoelektrischen Moduls, um die Flexibilität des Gurtes 12 im Bereich des Temperaturübertragungsbereichs A zu gewährleisten. Des Weiteren wurde festgestellt, dass mehrere kleine in Bezug auf die Breite gesehen über den Temperaturübertragungsbereich A verteilte thermoelektrische Module 14a-n die Flexibilität des Gurtes steigern, ohne dabei die Übertragung der Wärmeenergie auf die Körperstelle zu verringern.

Vorzugsweise ist die Breite B des Lüfters 16a-n in Bezug auf die Umfangsrichtung UR des Gurtes 12 um -5% bis 50%, vorzugsweise -5% bis 25%, besonders bevorzugt -5% bis 5% im Vergleich zu der Breite B des thermoelektrischen Moduls 14a-n dimensioniert.

Besonders bevorzugt sind die Mittelpunkte des thermoelektrischen Moduls 14a-n und des Lüfters 16a-n im Wesentlichen auf einer Gerade G zueinander ausgerichtet.

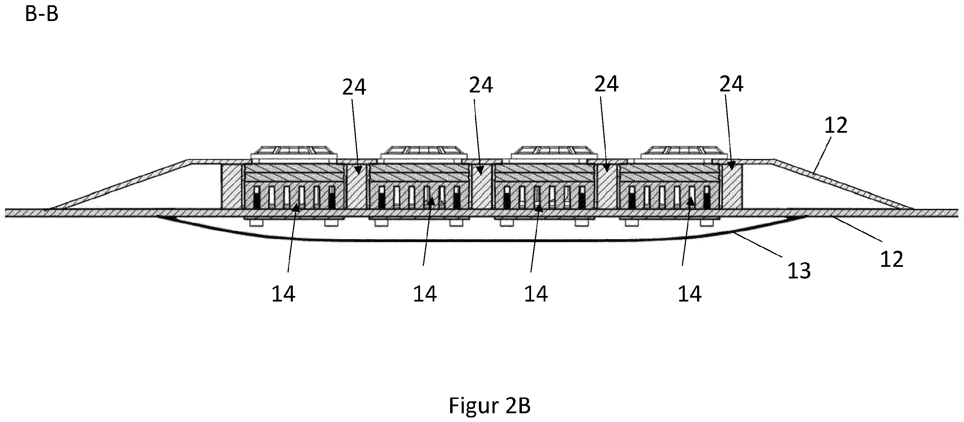

In dem hier gezeigten Beispiel sind eine Reihe von thermoelektrischen Modulen 14a-n und Lüfter 16a-n mit jeweiligen Auslassöffnungen 18a-n entlang der Umfangsrichtung UR des Gurtes 12 in einem Temperaturübertragungsbereich A in einem Abstand D von mindestens 0,1cm bis höchstens 10cm, vorzugsweise 1cm - 3cm angeordnet. Vorzugsweise sind die Reihen von thermoelektrischen Modulen 14a-n und Lüfter 16a-n mit jeweiligen Auslassöffnungen 18a-n parallel zueinander ausgerichtet, wobei Luftstromleitplanken 24 (siehe Figur 2B) zwischen den Reihen von thermoelektrischen Modulen 14a-n und Lüfter 16a-n mit jeweiligen Auslassöffnungen 18a-n und der ersten Lage 12a und der zweiten Lage 12b angeordnet sind. Die Luftstromleitplanken 24 sind derart an die erste Lage 12a und die zweite Lage 12b befestigt, sodass diese einen Luftstromkanal quer zur Umfangrichtung in Richtung des jeweiligen Lüfters 16a-n und der jeweiligen Auslassöffnung 18a-n zwischen der ersten Lage 12a und der zweiten Lage 12b bilden.

Figur 1B zeigt die Vorrichtung von der Rückseite beziehungsweise von der Seite, die nicht zur Körperstelle zeigt, beziehungsweise von der Körperstelle abgewandt ist.

Wie hier gezeigt, sind Auslassöffnungen 18a-n vorgesehen, die den Luftstrom welcher das thermoelektrische Modul kühlt, nach außen leiten. Diese Auslassöffnungen 18a-n sind in bevorzugter Weise ebenfalls auf der gleichen Gerade G angeordnet wie das thermoelektrische Modul 14a-n und der Lüfter 16a-n. Beispielsweise kann die jeweilige Auslassöffnung 18a-n in der zweiten Lage 12b angeordnet sein, so dass die Abluft über die Seite, die der Körperstelle abgewandt ist, nach außen geführt wird. Alternativ können die Auslasseröffnungen 18a-n zwischen der ersten Lage 12a und der zweiten Lage 12b (nicht gezeigt) angeordnet sein. Dies hat den Vorteil, dass die Abluft nicht durch äußere Mittel, wie zum Beispiel Kleidung, teilweise verschlossen wird. Dadurch kann ein Tragen der Vorrichtung 10 auch im Alltag unter der Kleidung gewährleistet werden.

Der zumindest eine Lüfter 16a-n kann alternativ auch zwischen der ersten Lage 12a und der zweiten Lage 12b als ein Axiallüfter ausgestaltet sein, so dass der Einlass des Axiallüfters zwischen der ersten Lage 12a und der zweiten Lage 12b liegt. Dies hat ebenfalls den Vorteil, dass die Zuluft für den Lüfter 16a-n nicht durch externe Mittel, wie zum Beispiel Kleidung, teilweise verschlossen wird.

Figur 1C zeigt die Vorrichtung in einer Seitenansicht. Die erste Lage 12a ist teilweise über der zweiten Lage 12b angeordnet. Die erste Lage 12a und/ oder die zweite Lage 12b sind derart verbunden, beispielsweise zusammengenäht, dass sie eine Stirnseite bilden. In einem Beispiel können die Einlassöffnungen und die Auslassöffnungen an der Stirnseite angeordnet sein (nicht gezeigt). Wie hier gezeigt, können die Einlassöffnungen des Lüfters 16a-n an der ersten und der zweiten Lage angeordnet sein. Dies gewährleistet eine maximale Luftromerzeugung des Lüfters 16a-n.

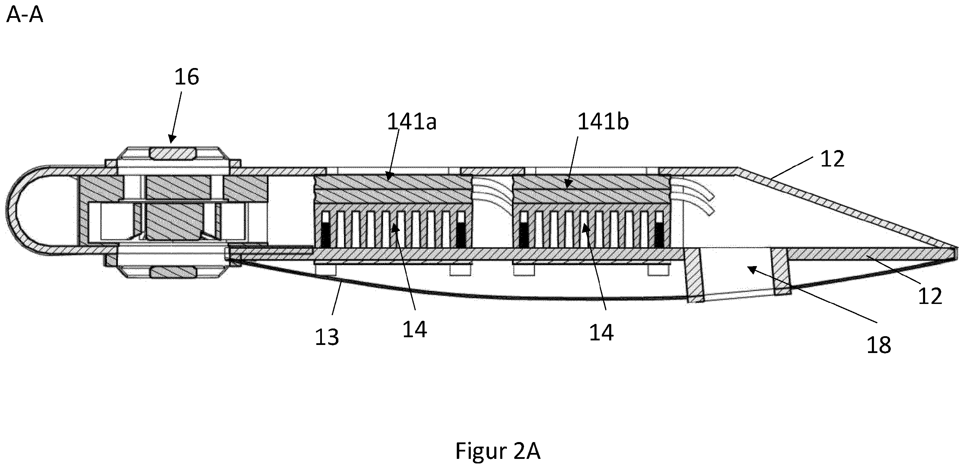

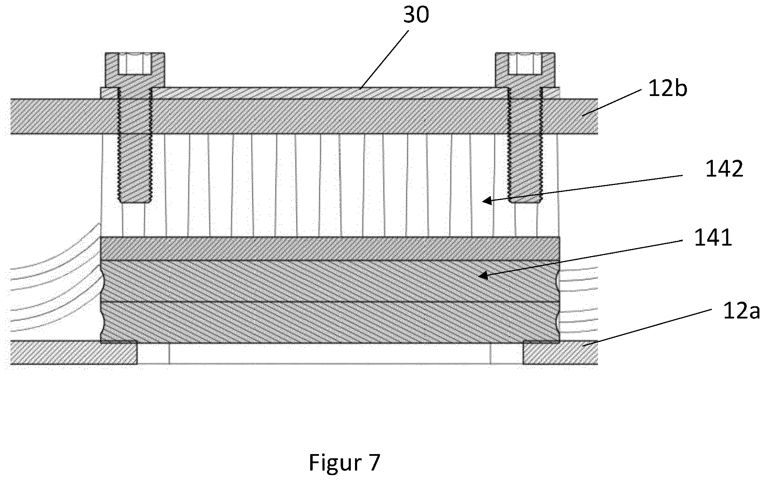

Figur 2A zeigt eine Schnittansicht A-A quer zur Umfangsrichtung UR. Wie beispielhaft gezeigt, weist das thermoelektrische Modul 14, zwei Peltierelemente auf 141a, 141b auf. Die thermoelektrischen Module sind über Kabel mit der Kontrolleinrichtung 28 (nicht gezeigt) verbunden. Die thermoelektrischen Module 14a-n sind über die Rückseite an der zweiten Lage 12b verbunden. Vorzugsweise sind die thermoelektrischen Module 14a-n über eine Platte 30 (siehe auch Figur 7). Die Schrauben können beispielsweise direkt in die Wärmesenke 142 über selbstschneidende Schrauben eingeschraubt werden. Die Oberseite des thermoelektrischen Elements steht mit der Körpestelle direkt in Kontakt und ist leicht versetzt oder bündig mit der ersten Lage 12a ausgestaltet.

Wie hier weiterhin beispielhaft gezeigt, ist eine Auslassöffnung 18 an der zweiten Lage 12b vorgesehen. In einem Beispiel kann die Rückseite der Vorrichtung 10 über eine Abdecklage 13 verfügen, wobei eine Öffnung mit der Auslassöffnung 18 der zweiten Lage 12b bereitgestellt ist.

Wie in Figur 2 gezeigt, ist Lüfter 16a-n, vorzugsweise ein Radiallüfter, derart ausgerichtet, um einen Luftstrom zwischen der ersten 12a und zweiten Lage 12b zu erzeugen.

Wie in Figur 2B beispielhaft gezeigt weisen die erste Lage 12a und/ oder die zweite Lage 12b des Gurtes 12 Ausnehmungen auf, um das thermoelektrische Modul 14a-n und/ oder den Lüfter 16a-n an den Gurt (12) anzuordnen. Dadurch kann insbesondere die Oberseite des thermoelektrischen Elements 141 in direkten Kontakt mit der zu behandelnden Körperstelle treten, sodass die Wärmeenergie des thermoelektrischen Elements 141, vorzugsweise Peltierlements, direkt auf die Körperstelle übertragen werden kann und muss nicht erst durch die Gewebeschicht der ersten Lage 12a diffundieren.

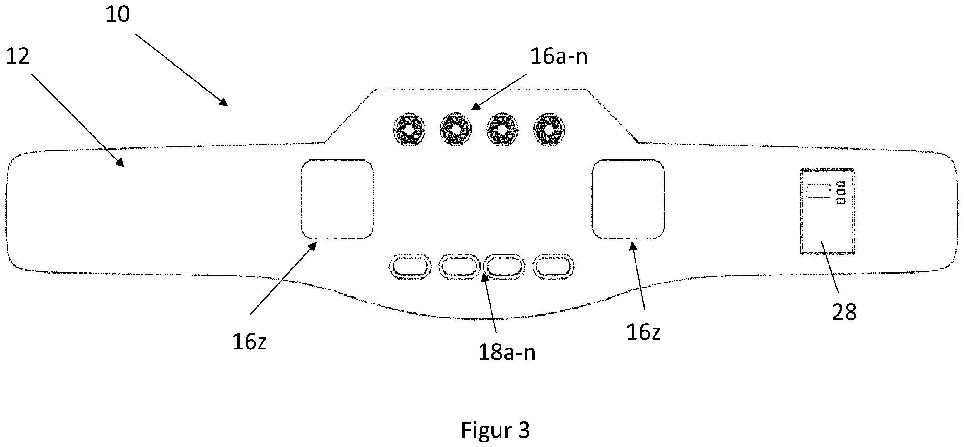

Wie in Figur 3 gezeigt kann die Kühlung des thermoelektrischen Elements 141 weiter erhöht werden, indem mindestens ein weiterer Lüfter 16z an jeweils einem Ende des Temperaturübertragungsbereich A entlang der Umfangsrichtung UR angeordnet ist.

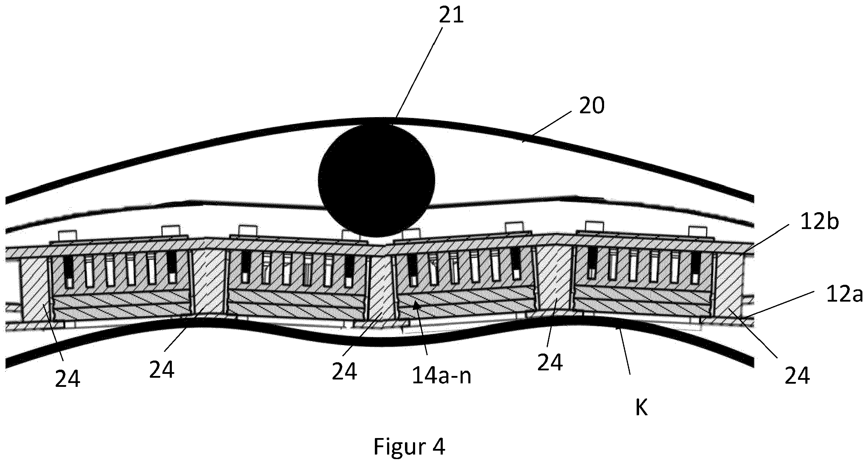

Figur 4 zeigt ferner beispielhaft eine Spannvorrichtung 20, sodass der Gurt 12 zumindest im Temperaturübertragungsbereich A an die Körperstelle anpressbar ist. Wie hier beispielhaft gezeigt, weist die Spannvorrichtung 20 Distanzelemente 21 auf, um den Anpressdruck entlang des Temperaturübertragungsbereichs A an einer Körperstelle K anzupassen. Weiterhin ist hier beispielsweise gezeigt, dass die flexiblen Luftstromleitplanken 24 derart flexibel ausgestaltet sind, dass sich diese an die Kontur der zu behandelnden Körperstelle anpassen. Dadurch kann die Kühlung der Vorrichtung verbessert werden ohne dabei an Flexibilität für den Gurt einzubüßen.

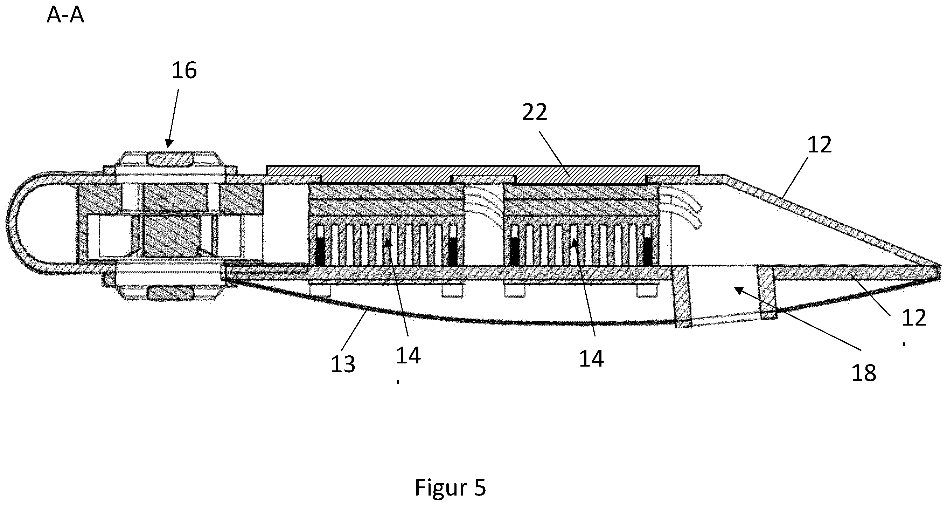

In einem weiteren Beispiel, siehe Figur 5, kann die Vorrichtung 10 eine Temperaturverteilerlage 22 aufweisen, welche an einer der Körperstelle zugewandten Seite der ersten Lage 12a des Gurtes 12 befestigbar ist. Dies ermöglicht eine breite und gleichmäßige Verteilung der Wärmeenergie des thermoelektrischen Moduls auf die Temperaturverteilerlage und somit die zu behandelnde Körperstelle. Die Temperaturverteilerlage 22 kann auch als flexible Wärmeübertragungsschicht bezeichnet werden und kann aus einem wärmeleitenden Polymer z.B. Wärmeleitsilikon, oder wärmeleitenden TPU bestehen.

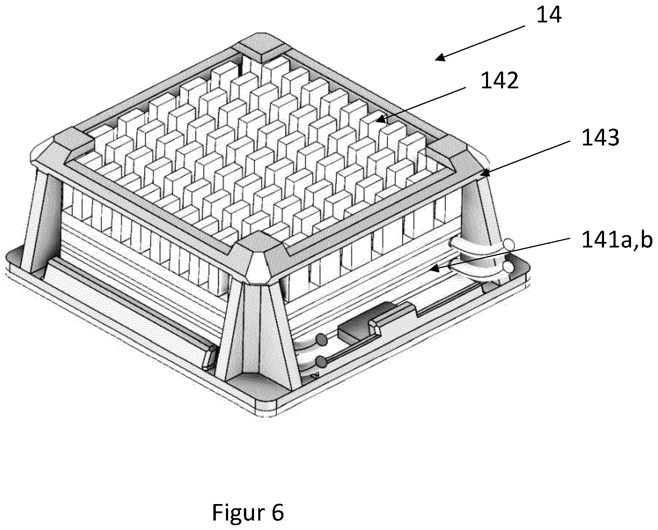

Figur 6 zeigt eine perspektivische Ansicht des thermoelektrischen Moduls 14 aufweisend ein thermoelektrisches Element 141, einen Kühlkörper 142 und einen Rahmen 143. Das thermoelektrische Element 141 kann wie hier beispielhaft gezeigt mindestens zwei Peltierelemente 141a, 141b umfassen. Des Weiteren kann das thermoelektrische Modul 14 ferner eine Wärmesenke 142 aufweisen, welche durch einen Rahmen 143 an das thermoelektrische Element 141 fixiert ist.

In einem weiteren Beispiel, siehe Figur 7, weist die Vorrichtung eine Platte 30 auf, welche auf die zweite Lage 12b von außen aufgebracht ist und über eine Schraubverbindung, vorzugsweise selbstschneidende Schrauben, mit der Wärmesenke 142 des thermoelektrischen Moduls verbunden ist.

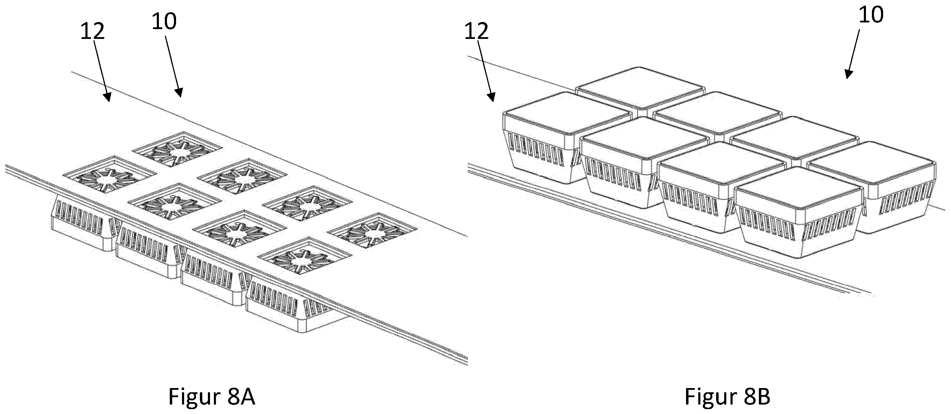

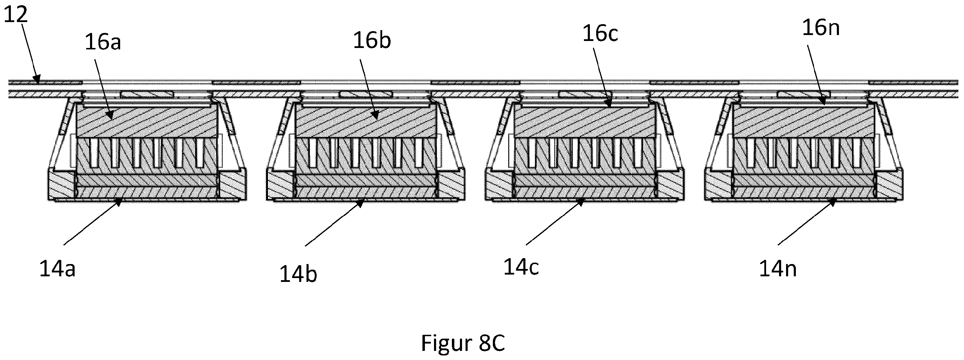

In einer alternativen Ausführungsform (siehe Figur 8A-C), ist das thermoelektrische Modul 14a-n und ein Axiallüfter miteinander verbunden und über die Rückseite des Axiallüfters an der Gurt 12 befestigt. In anderen Worten sind der Axiallüfter und das thermoelektrische Modul als eine Einheit ausgebildet und an den Gurt 12 wie eine Glocke befestigt. Dadurch hängen die thermoelektrische Module frei herunter und sind flexibel über eine Befestigung am Gurt schwenkbar. Dabei ist der Gurt an der Rückseite des Axiallüfters angeordnet und drückt somit die thermoelektrischen Module im gewickelten Zustand in effizienter Weise an die Körperstelle.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 10: Kalt- und Wärmevorrichtung
- 12: Gurt
- 12a: erste Lage
- 12b: zweite Lage
- 13: Abdecklage
- 14a-n: thermoelektrisches Modul
- 16a-n: Lüfter
- 18a-n: Auslassöffnungen
- 20: Spannvorrichtung
- 21: Distanzelemente
- 22: Temperaturverteilerlage
- 24: Luftleitplanken
- 26: Befestigungsbereich
- 28: Kontrolleinrichtung
- 30: Platte
- 141: thermoelektrisches Element
- 142: Wärmesenke
- 143: Rahmen
- A: Temperaturübertragungsbereich
- B: Breite
- D: Distanz
- UR: Umfangsrichtung
- G: Gerade

## Patentansprüche

1. Eine tragbare Kalt- und Wärmevorrichtung (10) zum Temperieren einer Körperstelle, aufweisend:
- einen Gurt (12) bestehend aus mindestens zwei im Wesentlichen zumindest teilweise übereinanderliegenden Lagen (12a, 12b);
- mindestens ein thermoelektrisches Modul (14a-n) aufweisend mindestens ein thermoelektrisches Element (141), vorzugsweise ein Peltierelement,
- mindestens einen Lüfter (16a-n), vorzugsweise einen Radiallüfter,
wobei der Gurt (12) eine erste Lage (12a), die der Körperstelle zugewandt ist, und eine zweite Lage (12b), die der Körperstelle abgewandt ist, aufweist;
**dadurch gekennzeichnet, dass**
das mindestens eine thermoelektrische Modul (14a-n) und/ oder der Lüfter (16a-n) zumindest an die erste Lage (12a) und/ oder zweite Lage (12b) befestigt sind;
wobei das thermoelektrische Modul (14a-n) und der Lüfter (16a-n) voneinander getrennt an dem Gurt (12) angeordnet sind.

2. Die Kalt- und Wärmevorrichtung (10) nach Anspruch 1, wobei der Lüfter (16a-n) derart ausgerichtet ist, um einen Luftstrom zwischen der ersten Lage (12a) und der zweiten Lage (12b) zu erzeugen.

3. Die Kalt- und Wärmevorrichtung (10) nach Anspruch 1 oder 2, wobei eine Auslassöffnung (18a-n) luftstromabwärts angeordnet ist, wobei die Auslassöffnung (18a-n) in der zweiten Lage (12b) oder zwischen der ersten Lage (12a) und zweiten Lage (12b) angeordnet ist.

4. Kalt- und Wärmevorrichtung (10) nach einem der vorhergehenden Ansprüche, wobei die Breite (B) des Lüfters (16a-n) in Bezug auf die Umfangsrichtung (UR) des Gurtes (12) um - 5% bis +50%, vorzugsweise -5% bis +25%, besonders bevorzugt -5% bis +5% im Vergleich zu der Breite (B) des thermoelektrischen Moduls (14a-n) dimensioniert ist.

5. Kalt- und Wärmevorrichtung (10) nach einem der vorhergehenden Ansprüche, wobei die Mittelpunkte des thermoelektrischen Moduls (14a-n) und des Lüfters (16a-n) im Wesentlichen auf einer Gerade (G) zueinander ausgerichtet sind.

6. Kalt- und Wärmevorrichtung (10) nach einem der vorhergehenden Ansprüche, wobei die erste Lage (12a) und/ oder die zweite Lage (12b) des Gurtes (12) Ausnehmungen aufweisen, um das thermoelektrische Modul (14a-n) und/ oder den Lüfter (16a-n) an den Gurt (12) anzuordnen.

7. Die Kalt- und Wärmevorrichtung (10) nach einem der vorhergehenden Ansprüche, wobei eine Reihe von thermoelektrischen Modulen (14a-n) und Lüfter (16a-n) mit jeweiligen Auslassöffnungen (18a-n) entlang der Umfangsrichtung (UR) des Gurtes (12) in einem Temperaturübertragungsbereich (A) in einem Abstand von mindestens 0,1cm bis höchstens 10cm, vorzugsweise 1cm - 3cm angeordnet sind.

8. Die Kalt- und Wärmevorrichtung nach einem der vorhergehenden Ansprüche, wobei mindestens ein weiterer Lüfter (16z) an jeweils einem Ende des Temperaturübertragungsbereich (A) entlang der Umfangsrichtung (UR) angeordnet sind.

9. Die Kalt- und Wärmevorrichtung (10) nach einem der vorhergehenden Ansprüche, ferner aufweisend eine Spannvorrichtung (20), sodass der Gurt (12) zumindest im Temperaturübertragungsbereich (A) an die Körperstelle anpressbar ist.

10. Die Kalt- und Wärmevorrichtung (10) nach Anspruch 9, wobei die Spannvorrichtung (20) Distanzelemente (21) aufweist, um den Anpressdruck entlang des Gurtes (12) anzupassen.

11. Die Kalt- und Wärmevorrichtung (10) nach einem der vorhergehenden Ansprüche, ferner aufweisend eine Temperaturverteilerlage (22), welche an einer der Körperstelle zugewandten Seite der ersten Lage (12a) des Gurtes (12) befestigbar ist.

12. Die Kalt- und Wärmevorrichtung (10) nach einem der vorhergehenden Ansprüche, wobei das thermoelektrische Element (141) mindestens zwei Peltierelemente (141a, 141b) umfasst.

13. Die Kalt- und Wärmevorrichtung (10) nach einem der vorhergehenden Ansprüche, wobei das thermoelektrische Modul (14a-n) ferner eine Wärmesenke (142) aufweist, welche durch einen Rahmen (143) an das thermoelektrische Element fixiert ist.

14. Die Kalt- und Wärmevorrichtung (10) nach einem der vorhergehenden Ansprüche, ferner aufweisend eine Platte (30), welche auf die zweite Lage von außen aufgebracht ist und über eine Schraubverbindung mit der Wärmesenke des thermoelektrischen Moduls verbunden ist.

15. Die Kalt- und Wärmevorrichtung (10) nach einem der vorhergehenden Ansprüche, ferner aufweisend flexible Luftstromleitplanken (24), welche einen Luftstromkanal vom Lüfter zum thermoelektrischen Modul bilden.
